# EUROPEAN PATENT APPLICATION

(11) **EP 4 760 730 A1**
(43) Date of publication of application: **17.06.2026**
(21) Application number: 24315566.0
(22) Date of filing: 10.12.2024
(51) Int. Cl.: G16H 10/40, G16H 20/10, G16H 20/60, G16H 50/30

(54) **SYSTEM AND METHOD FOR IMPROVING RESCUE CARBOHYDRATE RECOMMENDATIONS IN DIABETES MANAGEMENT**

(71) Applicant: Diabeloop, 38000 Grenoble (FR)
(72) Inventor: Lachal, Sylvain, 38600 Fontaine (FR); Romero-Ugalde, Hector, 38420 Le Versoud (FR); Desir, Chesner, 38000 Grenoble (FR); Huneker, Erik, 38120 Saint-Egreve (FR)
(74) Representative: Lannel, Pierre Olivier

(57) **Abstract**

Control device (30) for determining an amount of carbohydrate (RC) recommendation. The control device comprises a retrieving unit (32), the retrieving unit (32) being configured to retrieve user data, an outlier unit (34), the outlier unit (34) being configured to determine whether a user data is considered to be an outlier or not, an RC unit (36), the RC unit (36) being configured to estimate an RC recommendation using mathematical functions of the user data; and a transmission unit (38), the transmission unit (38) being configured to transmit the RC recommendation. The transmission unit (38) is configured to transmit the RC recommendation if and only if the last user data is not considered to be an outlier.

## Description

### FIELD OF THE INVENTION

The present invention relates to the field of diabetes management, specifically to a system and method for improving the accuracy and relevance of rescue carbohydrate recommendations.

### BACKGROUND OF THE INVENTION

In the field of diabetes management, the use of automated insulin delivery devices and related software applications has become increasingly prevalent. These systems often rely on continuous glucose monitoring (CGM) to provide real-time data on blood glucose levels, which is then used to calculate and administer appropriate insulin doses. A particular challenge in this field is the management of hypoglycemia, a condition characterized by abnormally low blood glucose levels. To counteract hypoglycemia, individuals with diabetes may be advised to consume additional carbohydrates, often referred to as rescue carbohydrates (RC). The recommendation of RC is typically based on current and predicted blood glucose levels, as well as other factors such as insulin on board (IOB), which refers to the amount of insulin that has been administered and is still available within the user's body.

However, the accuracy and relevance of RC recommendations can be influenced by various factors, including the reliability of the CGM data and the algorithms used to interpret this data. For instance, CGM sensors can be subject to outliers, which are data points that deviate markedly from other observations in a dataset. Outliers may arise due to variability in the measurement or may indicate error. Outliers can occur for many reasons, such as a change in device behavior or environmental factors.

Furthermore, the dynamics of blood glucose levels can be complex and influenced by various physiological and lifestyle factors. For instance, physical activity can increase insulin sensitivity and glucose uptake by muscles, potentially increasing the risk of exercise-induced hypoglycemia. Therefore, the ability to accurately predict future blood glucose levels and adjust RC recommendations accordingly is a challenging aspect of diabetes management.

In addition, the calculation of RC recommendations often involves the use of mathematical models and algorithms that take into account various parameters. However, the precision and reliability of these models can be influenced by the accuracy of the input parameters and the inherent variability in the user's physiological responses.

Despite the advancements in diabetes management technologies, there remains a deficiency in the prior art in terms of optimizing the accuracy and relevance of RC recommendations in various contexts. Existing systems often fall short in effectively identifying and handling outliers in CGM data, accurately predicting future blood glucose levels, and dynamically adjusting RC recommendations based on real-time data and individual user characteristics. Therefore, there is a substantial unmet demand for a system and method that can enhance the management of hypoglycemia through improved RC recommendations, while maintaining a design that is both user-friendly and amenable to integration with existing diabetes management infrastructure.

Document EP4268719A1 relates to an electronic device that includes a medicament delivery unit for delivering medication to the user and a biological sensor for sensing one or more analytes or biological data of the user and generating biological sensor data indicative of the sensed biological data. The electronic device further includes a compression sensor for sensing compression of the biological sensor and/or biological tissue of the user and generating compression sensor data. However, such a compression sensor can fail, has a weight and consumes energy. Therefore, the compression sensor can become a burden for the user.

The present system and method aim to address the technical problems in the prior art by improving the relevance and accuracy of rescue carbohydrate (RC) alerts in various contexts.

The invention thus aims to answer at least partially the above presented technical problems.

### BRIEF SUMMARY OF THE INVENTION

The present disclosure relates to a system and method for enhancing the relevance and accuracy of rescue carbohydrate (RC) alerts in the context of diabetes management. More specifically, the disclosed system and method aim to address the technical challenges associated with the accurate prediction and recommendation of RC in various scenarios, such as during periods of physical activity, postprandial periods, and during sleep when compression artifacts may occur. The system and method disclosed herein utilize a combination of user data, including insulin infusion amounts, ingested carbohydrates, and physiological values such as measured blood glucose levels or interstitial blood glucose levels, to determine RC recommendations. The system further incorporates an outlier unit to identify and exclude anomalous blood glucose readings, thereby improving the accuracy of the RC recommendations. The RC unit of the system estimates the RC recommendation using mathematical functions, such as slopes for example, of the measured blood or interstitial glucose levels, providing a dynamic assessment of the user's glucose trends. This enables the system to anticipate potential hypoglycemic events and recommend appropriate interventions in a timely manner. The system's ability to accurately estimate and recommend an appropriate amount of RC is a proactive measure to prevent severe hypoglycemia and ensure the user's safety. The system's ability to transmit the RC recommendation to another device enables the user or healthcare provider to receive timely alerts and take appropriate action, thereby enhancing the user's safety and the efficacy of diabetes management. The disclosed system and method thus provide a comprehensive and personalized approach to managing RC alerts, addressing the technical challenges in the prior art and enhancing the safety and efficacy of diabetes management.

Therefore, the invention relates A control device for determining an amount of RC recommendation, the control device comprising:
- a retrieving unit, the retrieving unit being configured to retrieve user data, each data of the user data having a timestamp and the user data being related to a unique user, the user data comprising at least:
   ∘ an amount of insulin infused to the unique user;
   ∘ an amount of carbohydrates ingested by the unique user;
   ∘ a plurality of physiological values of the unique user, the plurality of physiological values of the unique user comprising at least measured blood glucose levels;
- an outlier unit, the outlier unit being configured to determine whether measured blood glucose level of the measured blood glucose level is considered to be an outlier or not;
- an RC unit, the RC unit being configured to estimate an RC recommendation using mathematical functions of the measured blood glucose levels; and
- a transmission unit, the transmission unit being configured to transmit the RC recommendation;
wherein the transmission unit is configured to transmit the RC recommendation if and only if the last measured blood glucose level of the measured blood glucose levels is not considered to be an outlier.

According to the present invention, "measured blood glucose levels" means glucose level measured in blood or glucose level measured in interstitial tissue.

The outlier unit's capability to determine whether a measured blood glucose level is an outlier or not allows the system to disregard anomalous readings that could reduce the RC recommendation accuracy.

The RC unit's function of estimating an RC recommendation using slopes of the measured blood glucose levels allows a dynamic assessment of the user's glucose trends. This enables the system to anticipate potential hypoglycemic events and recommend appropriate interventions in a timely manner, potentially reducing the risk of severe hypoglycemia for example.

The transmission unit's conditional operation, which transmits the RC recommendation if and only if the last measured blood glucose level is not considered to be an outlier, ensures that the recommendations provided to the user are based on reliable and accurate data. This selective transmission acts as a safeguard against providing potentially harmful advice based on erroneous readings.

In the context of the present disclosure, "carbohydrates" (RC) refers to a carbohydrate quantity of carbohydrate intake recommended to a user to counteract an anticipated or occurring hypoglycemic event. The RC recommendation is calculated to raise the user's blood glucose level to a safe range, thereby providing a quick source of glucose that can be readily absorbed and utilized by the body. The RC recommendation is particularly useful for individuals with diabetes who are at risk of hypoglycemia due to insulin therapy or other factors affecting blood glucose levels. The system's ability to accurately estimate and recommend an appropriate amount of RC is a proactive measure to prevent severe hypoglycemia and ensure the user's safety.

In the context of the present disclosure, an "outlier" refers to a data point that deviates markedly from other observations in a dataset. Outliers may arise due to variability in the measurement or may indicate error; the latter are sometimes excluded from the data set. Outliers can occur for many reasons, such as a change in device behavior or environmental factors such as a compression of a Continuous Glucose Monitor (CGM) if the measured blood glucose levels are measured by a CGM. The ability to identify and handle outliers is particularly useful in systems that rely on accurate data to provide recommendations or alerts, as it helps to ensure that such recommendations or alerts are based on reliable and representative data.

In a particular embodiment, the control device comprises an alert unit configured to create an alert based on the RC recommendation. Such a configuration allows the control device to alert the user and therefore reduce the health risks of hypoglycemia. The alert must be of any kind such as a visual alert, audible alert or haptic alert for example.

In some aspects, the transmission unit may be configured to transmit the RC recommendation to another device, such as a mobile phone, a smartwatch, a dedicated receiver, or a healthcare provider's monitoring system. This transmission can occur over various communication protocols, including but not limited to Bluetooth, Wi-Fi, cellular networks, or near-field communication (NFC). The ability to transmit the RC recommendation to another device enables the user or healthcare provider to receive timely alerts and take appropriate action, thereby enhancing the user's safety and the efficacy of diabetes management.

In some embodiments, the transmission unit may be configured to send an alert to the user or a designated recipient when the RC recommendation is transmitted. The alert may serve to notify the user or recipient of the RC recommendation, prompting them to take action based on the recommendation provided. The alert could be in the form of a visual notification, an audible alarm, a vibration, or any combination thereof, depending on the user's preferences and the capabilities of the receiving device. This feature enhances the responsiveness of the user or caregiver to potential hypoglycemic events, contributing to improved safety and proactive diabetes management.

According to the present invention, a measured blood glucose level is a blood glucose level measured on the unique user. The blood glucose level can be measured by any means such as a Continuous Glucose Monitor (CGM) or a Blood Glucose Monitor (BGM) for example.

According to the present invention an amount of carbohydrates ingested by the unique user corresponds to an amount of sugar ingested in a meal for example.

In some aspects, the outlier unit may be configured to determine outliers using any Bayesian method. Bayesian methods provide a probabilistic framework for updating beliefs about the state of a system based on new observations. This approach allows the outlier unit to incorporate prior knowledge about the expected behavior of blood glucose levels and continuously refine its understanding as new data becomes available. By utilizing Bayesian techniques, the outlier unit may be able to more accurately identify anomalous readings while accounting for the inherent variability in physiological measurements. This can lead to improved robustness in outlier detection across a wide range of scenarios and patient profiles.

According to an embodiment, the outlier unit is configured to determine outliers using a Kalman filter (KF).

An outlier unit configured to determine outliers using a KF allows the outlier unit to enhance the precision of identifying inaccurate measured blood glucose levels by effectively filtering out statistical noise and inaccuracies inherent in the data. This results in a more reliable dataset for generating RC recommendations, which is particularly beneficial for users who require precise and timely interventions to manage their blood glucose levels. The KF's predictive capabilities allow for continuous refinement of the system's understanding of the user's physiological state, thereby improving the overall accuracy and reliability of the RC recommendations provided by the control device.

In the context of the present disclosure, a "Kalman filter" (KF) is an algorithm that uses a series of measurements observed over time, containing statistical noise and other inaccuracies, and produces estimates of unknown variables that tend to be more accurate than those based on a single measurement alone or a single model alone.

According to an embodiment, the KF works in a two-step process: first, a prediction step estimates the current state of the system, and second, an update step refines the estimates by incorporating the latest measurement. The KF is particularly advantageous in the control device for determining outliers in measured blood glucose levels, as it can effectively filter out noise and, more notably, the analysis of the KF residuals-representing the prediction error with respect to observations-provides an indication of the concordance between measurements and model outputs. This analysis is especially useful for detecting sensor faults, as it allows for the identification of discrepancies that may suggest an outlier, thereby enhancing the reliability of the data set used for generating RC recommendations. For example, the KF may predict the system state by integrating a personalized insulin-to-glucose model, and then refine this prediction by comparing it to the actual measured blood glucose levels, thereby identifying any discrepancies that may indicate an outlier.

In some aspects, the outlier unit may employ various derivations of the Kalman Filter (KF) for the detection of candidate outliers in the measured blood glucose levels. These derivations could be, but are not limited to, Extended KF, Unscented KF, Cubature KF, Square Root KF, information KF, Ensemble KF, and Particle Filter for example. Each of these KF derivations offers a different approach to processing and analyzing the data, with some being more computationally intensive than others. For instance, the Particle Filter may require the propagation of many particles through the model equations to estimate posterior state distributions, which may be non-Gaussian, but it requires fewer assumptions regarding the linearity of the system and the statistical properties of the noise.

The outlier unit may also utilize various models, whether linear or nonlinear, physiological or empirical (blackbox, greybox), to assess the mismatch between the model predictions and the actual measured blood glucose levels (i.e. CGM measurements). This mismatch assessment is a core function of the outlier unit, as it helps to identify when the measured blood glucose levels deviate from expected patterns, which may indicate a fault or outlier of the measured blood glucose levels. Depending on the hardware capabilities and the specific requirements of the diabetes management system, a more parsimonious version of the KF, such as the base KF coupled with a linear model, may be preferred. This approach focuses on the ability of the method to capture sudden variations of mismatches between the model and the measurements, which is of paramount concern for sensor fault detection. In contrast, a more accurate state estimation, which might be desired for model-based control applications such as computing insulin from state prediction, would potentially require a more complex KF derivation. The choice of KF derivation and model type is thus informed by the trade-off between computational efficiency and the level of accuracy and robustness desired in the detection of outliers.

According to an embodiment, In order to enhance the accuracy of RC recommendations, the control device is configured to implement a three-step procedure for managing outliers in the measured blood glucose levels. Initially, the outlier unit is configured to use a (KF) to analyze the residuals of the blood glucose measurements level, thereby identifying candidate outliers that deviate from the predicted values. These candidates are then subjected to a series of data-driven confirmation rules, which may include assessing the temporal context of the readings, the physiological plausibility of the glucose trends, and the consistency of the data with known user activities or events. Upon confirmation of the outliers, a control strategy of the RC unit is refined accordingly. This may involve adjusting the RC recommendation to account for the identified outliers, ensuring that the system's response is based on the user's true glycemic status. By systematically determining, confirming, and responding to outliers, the control device provides a robust and reliable approach to managing RC alerts, thereby improving the safety and efficacy of diabetes management.

According to an embodiment, KF is configured to predict the system state by integrating a 6th order insulin-to-glucose linear model personalized from user's settings, the model parameters comprising at least:
- a meal ratio (MR); and/or
- an insulin sensitivity factor (ISF); and/or
- an insulin diffusion rate represented by a diffusion time constant (τ_{IOB}); and/or
- a meal digestion rate represented by a digestion time constant (τ_{D}).

Such a characteristic allows the control device to provide a tailored approach to diabetes management by incorporating individual user settings. This personalization ensures that the RC recommendations are based on a model that closely reflects the user's specific physiological response to insulin and carbohydrate intake, leading to more accurate and effective management of blood glucose levels. The use of a 6th order linear model allows for a nuanced representation of the insulin-to-glucose dynamics, which can account for complex interactions over time, thereby enhancing the predictive accuracy of the system. This can be particularly advantageous in preventing hypoglycemic events, as the system can more precisely forecast blood glucose trajectories and suggest timely and appropriate RC interventions.

In the context of the present disclosure, a "6th order insulin-to-glucose linear model" refers to a mathematical representation that characterizes the relationship between insulin administration and glucose levels over time, using a linear state space model approximating actual system dynamics. The model is described by a set of differential equations that represent the dynamics of insulin action and glucose absorption, up to the sixth derivative with respect to time. The 6th order model aims to capture the primary dynamics of the insulin-to-glucose interaction, providing a balance between model complexity and computational efficiency, which can be particularly useful for real-time applications in insulin delivery systems.

As stated above, the use of a 6th order linear model allows for a nuanced representation of the insulin-to-glucose dynamics, which can account for complex interactions over time, thereby enhancing the predictive accuracy of the system.

In the context of the present disclosure, "meal ratio" MR refers to a parameter used to personalize the insulin-to-glucose model, representing the ratio of the amount of carbohydrates in a meal to the amount of insulin administered to metabolize those carbohydrates. The MR is a factor in determining the insulin dosage for a given carbohydrate intake, and it is used to adjust the model parameters to reflect the user's individual dietary habits and insulin sensitivity. The MR can be set by the user, healthcare provider or determined based on historical data and may be adjusted over time to optimize glycemic control.

In the context of the present disclosure, "insulin sensitivity factor" (ISF) refers to a parameter that quantifies the relationship between insulin administration and its effect on lowering blood glucose levels. The ISF may be used to adjust the dosage of insulin to achieve a desired blood glucose target and is typically personalized based on the user's insulin sensitivity and metabolic response. The ISF can be determined through clinical assessment or derived from historical data of the user's insulin usage and corresponding blood glucose responses.

In the context of the present disclosure, "diffusion time constant" (τ_{IOB}) refers to a parameter representing the rate at which insulin diffuses into the bloodstream and begins to lower blood glucose levels after administration. This constant is an integral part of the insulin-to-glucose model, as it helps to predict the time-dependent effects of insulin on blood glucose concentration. The diffusion time constant can be personalized based on the user's physiological characteristics and may vary depending on various factors. By incorporating τ_{IOB} into the model, the control device can more accurately forecast the insulin's impact on blood glucose levels over time, which is particularly useful for timing RC recommendations to prevent or mitigate hypoglycemia.

In the context of the present disclosure, "digestion time constant" (τ_{D}) refers to a parameter that characterizes the rate at which ingested carbohydrates are digested and absorbed into the bloodstream as glucose and therefore appear in the measured blood glucose levels. This constant is a component of the insulin-to-glucose model that influences the timing and magnitude of the postprandial glucose response. The digestion time constant can be personalized to reflect the user's individual digestive kinetics, which may be influenced by factors such as meal composition, gastrointestinal motility, and metabolic health. By incorporating τ_{D} into the model, the control device can more accurately predict the rise in blood glucose levels following a meal, which is particularly useful for determining the timing and amount of insulin administration or RC recommendations to maintain glycemic control.

In the context of the present disclosure, the model parameters comprising at least a MR and/or a ISF and/or a τ_{IOB} and/or a τ_{D} means that the model comprises the parameters or derivatives of said parameters.

The synergistic integration of the meal ratio (MR), insulin sensitivity factor (ISF), insulin diffusion rate (τ_{IOB}), and meal digestion rate (τ_{D}) within the 6th order insulin-to-glucose linear model facilitates a comprehensive and individualized management of blood glucose levels. By combining these parameters, the control device can more accurately simulate the complex interplay between carbohydrate intake, insulin administration, and their respective rates of action and absorption. This synergy allows for the refinement of the model to reflect the user's specific metabolic patterns, enhancing the precision of the RC recommendations. The combined effect of these parameters ensures that the RC recommendations are not just based on static measurements, but are dynamically adjusted to the user's personalized physiological responses, leading to a more effective and proactive approach to preventing hypoglycemic events.

According to an embodiment, the outlier unit is further configured to consider the last measured blood glucose level of the measured blood glucose levels to be an outlier if a distance (d) involving an innovation matrix and KF residuals, is inferior to a dynamic threshold (d_{THR}).

Such a characteristic allows the control device to dynamically adjust the sensitivity of outlier detection, which is particularly beneficial in scenarios where blood glucose levels may fluctuate rapidly, such as during physical activity or after meal intake. By setting a dynamic threshold (d_{THR}) that adapts to the current physiological context of the user, the system can more accurately discern between true outliers and legitimate rapid changes in glucose levels. This results in a more robust and reliable RC recommendation process, minimizing the risk of false alerts and ensuring that interventions are based on the true state of the user's glycemic status. The ability to fine-tune outlier detection in real-time enhances the system's utility across a wide range of situations, providing users with a higher degree of confidence in the RC recommendations received.

In the context of the present disclosure, "distance" (d) refers to a metric used to quantify the discrepancy between the predicted state of a system and the actual observed measurements. In other words, d is a measure of how the KF prediction error varies from the expected prediction error. Therefore, the innovation matrix is used to account for the usual variability on the prediction error. An important distance d means that the KF unusually wrongly predicts, which can be symptomatic of a sensor fault. Specifically, it is the Mahalanobis distance, which is a multivariate distance measure that accounts for the correlations among the variables and scales the distance calculation according to the variability of each variable. This distance is calculated from an innovation matrix, which represents the variability on the (KF) residuals between the predicted and observed measurements, and the KF residuals, which are the discrepancies between the actual measurements and the KF predictions. If the calculated distance (d) is less than a dynamically determined threshold (d_{THR}), the last measured blood glucose level is considered to be an outlier. This approach allows for a more sophisticated outlier detection mechanism that is sensitive to the inherent variability and correlation structure of the data, leading to more accurate and reliable blood glucose level assessments for the purpose of RC recommendation.

In some embodiments, the dynamic threshold (d_{THR}) for outlier detection may be computed based on a moving mean or median of d signal values taken over a past window of time. This approach allows for the threshold to adapt to changes in the user's physiological state over time, providing a baseline that is reflective of the user's recent glycemic trends. To further enhance the accuracy of outlier detection, the computation of the moving mean or median may be trimmed to discard the extreme values, such as the peaks that may contain the sought outlier, thereby capturing a more representative baseline of d.

Additional embodiments may include applying an additive offset to d_{THR}, which can provide a safety margin to account for measurement uncertainties or expected physiological variations. Alternatively, a multiplicative scaling factor may be applied to d_{THR}, allowing the threshold to scale proportionally with the variability observed in d. This scaling can be particularly useful in situations where the measured blood glucose levels exhibit greater fluctuation, such as during periods of intense physical activity or stress, ensuring that the outlier detection remains sensitive to true outliers while avoiding false positives.

In some embodiments, the outlier unit may utilize a Statistical Chi-Square Test to assess the goodness of fit between KF residuals and a theoretical distribution, such as a Gaussian distribution. This test is particularly useful for determining whether the observed residuals from the KF deviate in a statistically meaningful way from what would be expected if the system were correctly modeling the underlying physiological process. A substantial deviation, as indicated by a Chi-Square Test result that exceeds a predefined threshold, may suggest that a measured blood glucose level is an outlier. This deviation is defined as reading a larger value on the residuals' distribution's expected value, or statistical mean, than is typically observed in the absence of outliers in the measured blood glucose levels. Such a finding would be indicative of a potential fault in the measured blood glucose levels or an anomaly in the glucose measurement process, prompting the outlier unit to potentially exclude the affected data point from the RC recommendation process.

In the context of the present disclosure, a "Statistical Chi-Square Test" is a statistical method used to compare observed data with data expected to be obtained according to a specific hypothesis. The test calculates the chi-square statistic, a measure of the discrepancy between observed and expected frequencies in one or more categories. This statistic is then compared to a chi-square distribution to determine the likelihood of the observed distribution occurring by chance. If the calculated chi-square statistic exceeds the value of the chi-square distribution for a given level of statistical confidence, it suggests that the observed data do not fit the expected distribution, indicating the presence of an outlier or a deviation from the hypothesized pattern.

In some embodiments, machine learning techniques may be employed to enhance the detection of outliers based on the residuals obtained from the Kalman filter. These techniques involve training machine learning models on historical data to classify normal and faulty behavior. Algorithms such as Support Vector Machines (SVM), Random Forests, or Neural Networks can be utilized for this purpose. SVMs offer effectiveness in high-dimensional spaces and their ability to model non-linear boundaries due to their kernel trick feature. Random Forests, which consist of multiple decision trees, offer robustness against overfitting and provide a measure of feature importances. Neural Networks, particularly deep learning models, are capable of learning complex patterns and relationships in the data through their layered structure.

The use of these machine learning techniques in classification and clustering contexts allows for a comprehensive analysis of the data, where each technique may capture different aspects or patterns indicative of outliers. By combining these techniques or running them concurrently, the system can leverage a consensus approach to enhance the reliability of outlier detection. For instance, a voting mechanism could be implemented where if a majority of the machine learning algorithms determine the presence of an outlier, the system may consider bypassing the RC recommendation.

Alternatively, a safer mode could require all algorithms to converge on the detection of an outlier before taking action. This multi-algorithm approach can improve the robustness of the system against false positives and negatives, ensuring that RC recommendations are based on the accurate classification of blood glucose level measurements.

According to an embodiment, the outlier unit is further configured to identify outliers specifically during a postprandial period.

Such a characteristic allows the control device to specifically target the postprandial period, which is a time when blood glucose levels can be particularly volatile, in an upward trend, due to the ingestion of food. By focusing on this period, the outlier unit can more effectively identify readings

(i.e. measured blood glucose levels) that do not conform to expected post-meal blood glucose patterns, thereby enhancing the accuracy of the RC recommendations. This targeted approach to outlier detection is especially beneficial for users who may experience unpredictable glycemic responses after meals, as it provides a more refined analysis of blood glucose data during a time when accurate RC recommendations are of utmost relevance for preventing postprandial hypoglycemia.

In some cases, during the postprandial period, particularly close to the time of meal declaration, the control device may adjust an anticipatory rule for predicting the RC recommendation. This adjustment is based on the expectation that glycemia will rise due to the meal declared to have been ingested. Consequently, the system can, in some instances, relax the usual anticipatory rule to avoid raising an RC alert that could disrupt the user unnecessarily. This relaxation is maintained up to a point where the user's glycemia (i.e measured blood glucose level) reaches a level below which an RC trigger is desired, ensuring that RC alerts are raised when truly warranted. This approach helps to prevent the overcorrection of blood glucose levels, which can be particularly disruptive post-meal when the body is naturally working to assimilate ingested carbohydrates and stabilize glycemia.

According to an embodiment, the RC unit is further configured to estimate an RC recommendation if the most recent measured blood glucose levels of the measured blood glucose levels show a deceleration.

Such a characteristic allows the control device to provide RC recommendations that are not just reactive to current blood glucose levels, but also predictive of future trends. By analyzing the deceleration in blood glucose levels, the RC unit can identify when the rate of decrease is slowing down, which may indicate an impending stabilization or potential reversal of the downward trend. This preemptive capability can be particularly advantageous for preventing hypoglycemia, as it allows for earlier intervention with RC when the blood glucose levels are still within a safe range, rather than waiting for them to reach a level that is already indicative of hypoglycemia. This proactive approach can improve the user's quality of life by reducing the frequency and severity of hypoglycemic episodes, and can also minimize the cognitive burden on the user by reducing the number of decisions they have to make regarding their glucose management.

According to an embodiment, the control device comprises a predictive unit, the predictive unit being configured to compute at least a predicted glycemia at least based on the user data. Such a characteristic allows the control device to enhance the management of diabetes by providing foresight into the user's glycemic future. The predictive unit's ability to compute predicted glycemia based on user data enables the system to forecast potential hypoglycemic events before they occur, allowing for preemptive measures to be taken. This predictive capability can lead to improved glycemic control, as it provides users and healthcare providers with valuable information to make informed decisions regarding carbohydrate intake and insulin dosing. Additionally, the predictive unit's use of user data for forecasting purposes ensures that the predictions are personalized and tailored to the individual's metabolic responses, further increasing the accuracy and relevance of the RC recommendations. This can result in a reduction of hypoglycemic episodes, enhanced patient safety, and an overall improvement in the quality of life for individuals with diabetes.

In the context of the present disclosure, a "predictive unit" refers to a component within the control device that is responsible for calculating future physiological states, such as predicted glycemia levels, based on current and historical user data. The predictive unit may utilize various mathematical models, algorithms, and data processing techniques to analyze trends and patterns in the user's physiological data, thereby enabling the system to anticipate future changes in blood glucose levels. For example, the predictive unit may employ a linear regression model to forecast blood glucose levels based on recent measurements and known rates of glucose absorption and insulin action. In another aspect, the predictive unit might use machine learning algorithms, such as neural networks or support vector machines, to analyze complex datasets and identify subtle correlations that can improve the accuracy of its predictions. Additionally, the predictive unit could incorporate time-series analysis to detect cyclical patterns in blood glucose fluctuations, which can be influenced by daily activities, meal times, and sleep cycles. These examples illustrate the versatility of the predictive unit in adapting to the individualized characteristics of the user's metabolic responses and lifestyle factors, thereby enhancing the personalized management of diabetes. In some embodiments, the predictive unit may also employ Model Predictive Control (MPC) strategies to enhance the prediction of future physiological states. MPC is a form of control algorithm that involves creating a model of the system to predict future outcomes and adjusting control inputs accordingly. In the context of glycemia management, MPC can be particularly useful for taking into account the delayed effect of insulin delivery and carbohydrate absorption on blood glucose levels. By considering a range of possible future scenarios and the likely impact of different insulin dosing strategies, the predictive unit can optimize the control of blood glucose levels over a specified prediction horizon, thereby contributing to more stable and precise diabetes management.

According to an embodiment, the RC unit is configured to estimate the RC recommendation if a predicted glycemia is less than a predicted glycemia threshold (θ1), the current glycemia is less than a current glycemia threshold (θ2), and an Insulin On Board (lOB) is greater than an IOB threshold (θ3).

Such a characteristic allows the control device to provide a more nuanced and responsive approach to managing blood glucose levels, particularly in the context of insulin therapy. By incorporating multiple thresholds that take into account predicted glycemia, current glycemia, and IOB, the RC unit can make more informed decisions about when to recommend carbohydrate intake. This multi-faceted analysis ensures that RC recommendations are made at the appropriate times, reducing the risk of over- or under-treatment of hypoglycemia. The integration of these thresholds allows for a more personalized and precise management of diabetes, which can lead to better overall glycemic control and a reduction in the incidence of hypoglycemic events. This proactive strategy can improve patient outcomes and may decrease the long-term complications associated with diabetes.

According to an embodiment, the RC unit is configured to dynamically adjust the predicted glycemia threshold (θ1), the current glycemia threshold (θ2), and the IOB threshold (θ3) based on real-time data and the user's current physiological state. For example, the thresholds may be adjusted to account for factors such as physical activity, stress, illness, or changes in medication. This dynamic adjustment allows the control device to provide RC recommendations that are tailored to the user's immediate situation, thereby enhancing the safety and efficacy of the insulin therapy.

Additionally, the RC unit may incorporate safety features that prevent the recommendation of RC when it is not indicated for safety or user's disturbance reason. For instance, if the predicted glycemia is above the predicted glycemia threshold (θ1) and the current glycemia is above the current glycemia threshold (θ2), the RC unit may withhold the RC recommendation even if the lOB is greater than the lOB threshold (θ3). This safety mechanism ensures that RC is recommended conservatively and in accordance with the user's actual glycemic status, minimizing the risk of unnecessary carbohydrate intake and potential subsequent hyperglycemia.

In the context of the present disclosure, "Insulin On Board" (IOB) refers to the amount of insulin that has been administered and is still available within the user's body. The IOB is a dynamic value that reflects the insulin that has yet to exert its full glucose-lowering effect. Estimating the lOB involves calculating the remaining available insulin from previous doses, taking into account the time since administration and the known pharmacokinetics of the insulin preparation used. The IOB can be influenced by factors such as the type of insulin, the dosage, and the individual's insulin sensitivity. For instance, rapid-acting insulin will have a different lOB profile compared to long-acting insulin. The lOB is an integral part of the decision-making process in insulin management, as it helps to prevent insulin stacking and the risk of hypoglycemia. In some embodiments, the control device is configured to estimate the lOB by utilizing a decay function that models the progressive absorption effect of insulin over time. This estimation allows the RC unit to adjust the RC recommendation by considering the residual impact of previously administered insulin on current and predicted glycemia levels.

According to an embodiment, a measured blood glucose level of the measured blood glucose levels is considered to be an outlier if it is considered by the outlier unit to be affected by a compression artifact.

Such a characteristic allows the control device to enhance the reliability of RC recommendations by identifying and excluding glucose measurements that may be distorted due to physical interference with the glucose monitoring device, wherein said glucose monitoring device is a CGM for example. Compression artifacts can occur when the sensor is subjected to pressure, leading to falsely low glucose readings that could trigger unnecessary RC recommendations. By recognizing these artifacts, the control device can prevent inappropriate responses to these inaccurate readings, thereby maintaining the accuracy of the RC recommendations and avoiding potential overtreatment of hypoglycemia. This feature is particularly beneficial for users who are active or sleep on their monitoring device for example, as it ensures that the RC recommendations are based on true glucose values rather than artifacts of the measurement process.

In the context of the present disclosure, a "compression artifact" refers to a type of error in glucose readings that occurs when an external force is applied to the glucose monitoring device, typically a Continuous Glucose Monitor (CGM), which results in a temporary deformation of the sensor or its surrounding tissue. This deformation can cause a disruption in the interstitial fluid dynamics or sensor function, leading to a transient and artificial change in the measured glucose level that does not accurately reflect the true blood glucose concentration. Compression artifacts are often characterized by a sudden, unphysiological drop in the recorded glucose levels, followed by a return to more typical readings once the pressure is relieved. These artifacts can be particularly problematic during periods of rest or sleep when the user may be lying on the sensor, or during intense physical activity where the device may be compressed against the body. The ability to accurately identify and exclude readings affected by compression artifacts is an integral part of ensuring the reliability of glucose monitoring systems and the safety of the user, as it helps to prevent inappropriate therapeutic decisions based on erroneous data.

In some aspects, the control device may utilize confirmation rules to verify that a KF outlier candidate is indeed an unphysiological compression artifact. The confirmation process may include a nighttime condition, which focuses on detecting nocturnal compression artifacts to reduce the risk of false positives that could be increased by day-to-day activities. The outlier unit may also be configured to consider the temporal distance to past declared, estimated, or measured physical activities and carbohydrate intakes, as these factors can influence blood glucose levels and the likelihood of compression artifacts.

A downward residual condition may be applied, where a negative residual value is a negative value which confirms a nadir trend for the outlying period. This condition is particularly relevant during nighttime, as it enhances the safety of detection by reducing the likelihood of false positives. However, this condition may be relaxed in other applications where nighttime detection is not as pertinent.

According to the present invention, a "nadir trend" refers to a pattern or trajectory of measured blood glucose levels reaching their lowest point over a given period.

The outlier unit may further be configured to evaluate the first derivative condition, where the current slope of the blood glucose level trend is compared to a predefined negative threshold. If the slope is less than this threshold, it confirms a substantial and unusual drop in glycemia, indicative of a potential compression artifact. Additionally, a second derivative condition may be assessed, where a sudden discontinuity in the slope, as compared to a predefined threshold, attests to an unphysiological change in trend.

An alternative embodiment for the second derivative condition may involve computing the ratio of two successive first derivative samples and comparing it to a predefined threshold. This approach allows for the detection of rapid changes in the rate of glycemia decrease, which are characteristic of compression artifacts.

Furthermore, a net IOB condition may be implemented, where the net IOB value is expected to be less than zero to enhance the safety of the detection process. This ensures that the sudden drop in measured blood glucose levels cannot be attributed to the effects of past insulin infusion. By incorporating these confirmation rules, the control device can more accurately identify and exclude compression artifacts, thereby improving the reliability of the RC recommendations and ensuring that interventions are based on accurate blood glucose measurements.

In the context of the present disclosure, "net Insulin On Board" or "net IOB" refers to the amount of insulin that has been administered and is still available within the user's body, adjusted for a basal insulin rate. The net lOB is calculated by subtracting the amount of insulin that would have been delivered as part of the user's basal rate from the total IOB. This calculation takes into account the insulin that has been infused to counteract ingested carbohydrates as well as insulin administered to correct high blood glucose levels. The net IOB provides a more accurate reflection of the insulin's potential effect on lowering blood glucose levels at any given moment, as it considers the insulin that is effectively in excess of the basal insulin requirements.

In some embodiments, the outlier unit of the control device is configured to analyze the relationships between various features, representing a feature space, such as measured blood glucose levels derivatives, net IOB, Carbohydrate On Board (COB), KF residuals, and additional sensor data like accelerometry or cardiac frequency for example, using a Support Vector Machine (SVM). The SVM allows one to make a hyperplane that effectively separates the feature space into two distinct categories: the outlying period and the physiological period. This separation is instrumental in distinguishing between normal physiological fluctuations and potential outliers that may indicate a fault in the measured blood glucose levels or an unphysiological event affecting the glucose readings.

In the context of the present disclosure, a "hyperplane" is a geometric concept that generalizes the notion of a plane in higher dimensions. Specifically, in a feature space of n dimensions, a hyperplane is a flat affine subspace of dimension n-1, which means it is one dimension less than the feature space itself. For example, in a three-dimensional space, a hyperplane would be a two-dimensional plane. In the context of machine learning and specifically in the use of Support Vector Machines (SVM), a hyperplane is used to separate different classes of data points by finding the plane that maximizes the margin between the classes. The hyperplane is defined by a set of weights and a bias, which are optimized during the training of the SVM to create the decision boundary that can classify new data points based on their position relative to the hyperplane.

In another embodiment, the outlier unit may utilize a trained Random Forest method to define a threshold surface within the feature space. The Random Forest method, which consists of an ensemble of decision trees, can classify the measured blood glucose levels as either outliers or non-outliers based on the aforementioned features. This method is particularly advantageous due to its ability to handle high-dimensional data and its robustness against overfitting, which is beneficial for the accurate detection of outliers in the measured blood glucose levels.

Further, in some cases, the outlier unit may implement Neural Networks, including Convolutional Neural Networks (CNNs) or Recurrent Neural Networks (RNNs), for the classification of outliers. These types of Neural Networks are adept at capturing complex patterns and temporal dependencies in the data, which can be particularly useful for identifying subtle or transient anomalies in the measured blood glucose levels that may not be readily apparent through other methods.

Additionally, the outlier unit may also incorporate Gaussian Process Regression (GPR) to model the relationship between input features and the output classification. GPR provides not just predictions for classification but also uncertainty estimates, which are of great value when defining a threshold surface with confidence intervals. These uncertainty estimates can enhance the decision-making process by providing a probabilistic assessment of the data, thereby allowing for more informed and cautious interventions in the management of blood glucose levels.

According to an embodiment, when a measured blood glucose level of the measured blood glucose levels is considered to be an outlier, the transmission unit is configured to not transmit RC recommendation until a bypass period. This bypass period is maintained until at least one of the measured blood glucose levels of the measured blood glucose levels has recovered and is not considered to be an outlier. The end of the bypass period is based on two conditions: either the time has reached or exceeded the nadir time plus a predetermined upward time, or the measured blood glucose level has risen to or above a certain level.

According to an embodiment, the bypass period is designed with safety exceptions to ensure unique user well-being. Should the most recently measured blood glucose level fall below a safety threshold, such as 55 mg/dL for example, or if the bypass period extends beyond a maximum duration, the transmission unit is configured to be able to transmit RC recommendation again.

Such a configuration allows the control device to prevent the risk of actual hypoglycemia that may not be detected due to a measured blood glucose level considered to be an outlier.

According to an embodiment, the control device is configured to assess a blood glucose level prediction at a time ahead of the bypass period, up to a predefined prediction horizon. If this blood glucose level prediction is below a predefined hypoglycemia safety threshold, the bypass period will be reduced to zero, as this indicates a genuine risk of hypoglycemia. Such a configuration allows the control device to prevent the risk of actual hypoglycemia. For the purpose of making these assessments, the control device may be configured to employ any suitable prediction means such as linear models, nonlinear models, or machine learning algorithms. The choice of prediction means can be tailored to the specific requirements of the diabetes management system, taking into account factors such as computational efficiency, prediction accuracy, and the user's individual physiological response patterns.

According to an embodiment, the outlier unit is configured to consider a measured blood glucose level to be affected by a compression artifact based at least on a deviation from a predicted glycemia and a measured blood glucose level.

Such a configuration allows the control device to discern between genuine fluctuations in blood glucose levels and those affected by compression artifacts. By comparing the deviation between predicted glycemia and actual measured blood glucose levels, the outlier unit can effectively identify when a reading is likely to have been compromised. This ensures that the RC recommendations are based on accurate and reliable data, particularly in situations where the CGM sensor might be under pressure, such as during sleep or physical activity. The ability to filter out these specific types of outliers can prevent unnecessary or incorrect carbohydrate intake recommendations, thereby optimizing the user's glycemic management and reducing the risk of hypoglycemia. This feature is especially advantageous for maintaining the integrity of the RC recommendation process, ensuring that users receive alerts and interventions that accurately reflect their physiological state.

In accordance with the present disclosure, the outlier unit of the control device is configured to utilize a predictive model that takes into account both the predicted glycemia and the actual measured blood glucose levels. The predictive model may employ various algorithms and techniques to forecast the expected glycemia based on a range of factors, including but not limited to, historical blood glucose data, recent trends in the user's blood glucose levels, and the user's physiological parameters. When the outlier unit detects a deviation between the predicted glycemia and the actual measured blood glucose level that exceeds a predefined threshold, it considers this an indication of a potential compression artifact. This threshold may be dynamically adjusted based on the user's current state and the variability of their blood glucose measurements. By setting this threshold, the control device can distinguish between normal physiological fluctuations in blood glucose levels and those readings that are likely to have been affected by external pressure on the glucose monitoring device. This feature is particularly useful in scenarios where the user is engaged in activities that may exert pressure on the sensor, such as sleeping or exercising. The ability to accurately identify and disregard these affected readings helps to maintain the integrity of the RC recommendation process, ensuring that the user is provided with reliable and actionable information for managing their diabetes.

According to an embodiment, the retrieving unit is further configured to retrieve user data including physical activity data of the user, and wherein the RC unit is configured to adjust the RC recommendation based on the physical activity data.

Such a configuration allows the control device to account for the impact of physical activity on blood glucose levels, which is a dynamic factor in diabetes management. The inclusion of physical activity data in the RC recommendation process enables the system to provide more personalized and context-aware recommendations. By adjusting the RC recommendation based on the user's physical activity, the system can better anticipate the physiological effects of exercise, which often include increased insulin sensitivity and glucose uptake by muscles, potentially increasing the risk of exercise-induced hypoglycemia. This feature enhances the system's ability to tailor RC recommendations to the user's lifestyle, promoting more effective and safer diabetes management. Additionally, it supports users in maintaining an active lifestyle by providing them with the confidence that their glucose levels are being monitored and managed with consideration of their physical activities.

According to an embodiment, the RC unit is configured to compute the RC recommendation based on a distance between a glycemia target and a linearly forecasted glycemia for a predefined prediction horizon, and wherein said distance is converted to carbohydrate units via a sugaring ratio.

Such a configuration allows the control device to provide a precise and personalized RC recommendation by quantifying the exact carbohydrate amount that is likely to bring the user's blood glucose level back to a desired target range. By calculating the distance between the glycemia target and the linearly forecasted glycemia, the RC unit can determine the magnitude of intervention that is appropriate for the specific situation. The conversion of this distance into carbohydrate units via a sugaring ratio simplifies the process for the user, translating complex data into actionable and understandable guidance. This approach not just aids in the immediate correction of hypoglycemia but also contributes to the long-term stability of blood glucose levels by providing tailored recommendations that are directly aligned with the user's physiological requirements. The ability to forecast glycemia and convert it into a tangible RC recommendation empowers users to manage their condition with greater confidence and precision, potentially reducing the frequency of hypoglycemic episodes and enhancing overall quality of life.

In the context of the present disclosure, a "sugaring ratio" is calculated based on the relationship between the amount of carbohydrates (CHO) that is expected to raise the blood glucose level to a target range and the anticipated increase in blood glucose level over a predefined prediction horizon. The sugaring ratio is estimated using a formula that takes into account the user's body weight (BW) and a standard absorption factor, which is typically derived from empirical data. For instance, the sugaring ratio may be estimated from the formula sugRatio = 20 BW / (0.6 80) in [gCHO/g/L], where BW is the body weight in kilograms. This ratio is then used to convert the distance between a predicted glycemia and a glycemia target into carbohydrate units, which represents the recommended amount of carbohydrates to be ingested to correct or prevent hypoglycemia. The sugaring ratio thus serves as a conversion factor that translates the anticipated change in blood glucose level into an equivalent amount of carbohydrates, facilitating the computation of a personalized RC recommendation.

According to an embodiment, the RC unit is further configured to adjust the RC recommendation based at least on IOB, such that an adjusted RC recommendation is an increasing function of the IOB.

Such a configuration allows the control device to dynamically tailor the RC recommendation in accordance with the amount of insulin that is still available within the user's body, known as Insulin On Board (lOB). By considering IOB, the RC unit can more accurately determine the amount of carbohydrates that the user may require to prevent or treat hypoglycemia. This is particularly advantageous as it accounts for the insulin's ongoing glucose-lowering effect, which can vary depending on factors such as the type of insulin used, the time since administration, and the user's insulin sensitivity. The ability to adjust the RC recommendation as an increasing function of the lOB ensures that the user is not advised to consume more carbohydrates than is actually necessary based on their current physiological state, thereby avoiding potential hyperglycemia. This feature enhances the precision of the RC recommendation, leading to more effective and safer blood glucose management, and ultimately contributes to the user's overall well-being and metabolic stability.

An RC recommendation based at least on lOB corresponds to a recommendation that is based on the net IOB, ensuring that the RC unit accounts for the dynamic insulin levels when determining the appropriate amount of rescue carbohydrates to recommend for preventing or treating hypoglycemia. This approach allows for a more personalized and precise RC recommendation, as it aligns the recommendation with the user's current physiological insulin profile.

In the context of the present disclosure, "basal insulin rate" refers to insulin administered to a user to maintain normal blood glucose levels in the absence of meals. This rate is typically set based on the user's basal metabolic insulin requirements, which are the insulin requirements during periods of fasting or in between meals. The basal insulin rate is designed to mimic the steady release of insulin by a healthy pancreas and is often delivered by an insulin pump or through long-acting insulin injections. The basal insulin rate can be adjusted based on various factors such as changes in the user's routine, physical activity, stress levels, or other physiological conditions that may affect insulin sensitivity. It is a foundational component of insulin therapy for individuals with diabetes, particularly those on intensive insulin therapy for example. The basal insulin rate is distinct from bolus insulin, which is administered to manage the rise in blood glucose levels due to carbohydrate intake from meals.

The invention also relates to a method for determining an amount of RC recommendation, the method being implemented by the control device as described above and comprising the steps of:
- retrieving the user data; determining the RC recommendation at least using mathematical functions of the measured blood glucose levels; and
- transmitting the RC recommendation;
wherein the step of transmitting the RC recommendation is executed if and only if the last measured blood glucose level of the measured blood glucose levels is not considered to be an outlier.

The embodiments, technical effects, and definitions disclosed herein with respect to the control device are also applicable to the method described herein. The method encompasses steps that fully utilize the functionalities and features of the control device described herein. Therefore, all embodiments, technical effects, and definitions pertaining to the device, including but not limited to the evolution of the ISF over time and how it is calculated, are equally applicable to the method. This ensures a comprehensive and unified understanding of both the control device and method aspects of the invention, facilitating the implementation and utilization of the disclosed technology across a range of applications.

The invention also relates to a computer program comprising instructions to cause the control device as described above to execute the steps of the method as described above.

Moreover, the disclosed system and method can be adapted for use in other applications within the healthcare industry where accurate and reliable prediction of physiological events and appropriate response recommendations are desired.

The invention also relates to a control system comprising an insulin infusion device, a CGM and control device comprising a recommendation unit and a retrieving unit.

While exemplary embodiments of the invention have been described, it will be understood by those skilled in the art that various changes, omissions and/or additions may be made, and equivalents may be substituted for elements thereof without departing from the spirit and scope of the invention. In addition, many modifications may be made to adapt a particular situation or material to the teachings of the invention without departing from the scope thereof. Therefore, it is intended that the invention is not limited to the particular embodiments disclosed for carrying out this invention, but that the invention will include all embodiments falling within the scope of the appended claims. Moreover, unless specifically stated any use of the terms first, second, etc. do not denote any order or importance, but rather the terms first, second, etc. are used to distinguish one element from another.

### BRIEF DESCRIPTION OF THE DRAWINGS

Embodiments of the invention will be described below with reference to the drawings, described briefly below:
- [Fig. 1] shows a system according to one embodiment of the invention; and
- [Fig. 2] shows a method for determining an amount of rescue carbohydrates according to one embodiment of the invention.

### DETAILED DESCRIPTION OF THE INVENTION

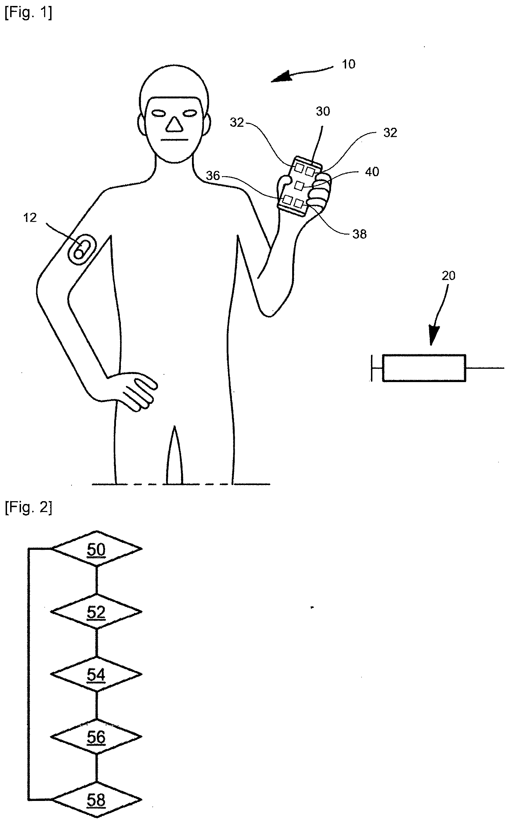

Figure 1 represents a system 10 comprising an insulin infusion device 20, a Continuous Glucose Monitor (CGM )12 and control device 30.

The control device 30 is configured to determine an amount of carbohydrate (RC) recommendation for a unique user and comprises a retrieving unit 32. The retrieving unit 32 is configured to retrieve user data, each data of the user data having a timestamp and the user data being related to the unique user. The user data comprises an amount of insulin infused to the unique user, an amount of carbohydrates ingested by the unique user, and a plurality of physiological values of the unique user, the plurality of physiological values of the unique user comprising at least measured blood glucose levels. The measured blood glucose levels are measured by the CGM 12.

In the context of the present disclosure, "carbohydrates" (RC) refers to a carbohydrate quantity of carbohydrate intake recommended to a user to counteract an anticipated or occurring hypoglycemic event. An RC can also be called a Rescue Carb. The RC recommendation is calculated to raise the user's blood glucose level to a safe range, thereby providing a quick source of glucose that can be readily absorbed and utilized by the body. The RC recommendation is particularly useful for individuals with diabetes who are at risk of hypoglycemia due to insulin therapy or other factors affecting blood glucose levels. The control device 30 ability to accurately estimate and recommend an appropriate amount of RC is a proactive measure to prevent severe hypoglycemia and ensure the user's safety.

According to the present invention, a measured blood glucose level is a blood glucose level measured on the unique user. The blood glucose level can be measured by any means such as a CGM as represented in figure 1 or a Blood Glucose Monitor (BGM) for example. An amount of carbohydrates ingested by the unique user corresponds to an amount of sugar ingested in a meal for example.

The retrieving unit 32 is further configured to retrieve user data including physical activity data of the user.

The control device 30 also comprises an outlier unit 34. The outlier unit 34 is configured to determine whether a measured blood glucose level of the measured blood glucose levels is considered to be an outlier or not. The outlier unit 34 capability to determine whether a measured blood glucose level is an outlier or not allows the control device 30 to disregard anomalous readings that could reduce the RC recommendation accuracy. In the context of the present disclosure, an "outlier" refers to a data point that deviates markedly from other observations in a dataset. Outliers may arise due to variability in the measurement or may indicate error; the latter are sometimes excluded from the data set. Outliers can occur for many reasons, such as a change in device behavior or environmental factors such as a compression of the CGM if the measured blood glucose levels are measured by a CGM. The ability to identify and handle outliers is particularly useful in systems that rely on accurate data to provide recommendations or alerts, as it helps to ensure that such recommendations or alerts are based on reliable and representative data.

The outlier unit 34 is configured to determine outliers using a Kalman filter (KF). In the context of the present disclosure, a "Kalman filter" (KF) is an algorithm that uses a series of measurements observed over time, containing statistical noise and other inaccuracies, and produces estimates of unknown variables that tend to be more accurate than those based on a single measurement alone or a single model alone. An outlier unit 34 configured to determine outliers using a KF allows the outlier unit 34 to enhance the precision of identifying inaccurate measured blood glucose levels by effectively filtering out statistical noise and inaccuracies inherent in the data. This results in a more reliable dataset for generating RC recommendations, which is particularly beneficial for users who require precise and timely interventions to manage their blood glucose levels. The KF's predictive capabilities allow for continuous refinement of a system's understanding of the user's physiological state, thereby improving the overall accuracy and reliability of the RC recommendations provided by the control device 30.

As represented in figure 2, the KF works in a two-step process: first, a prediction step 52 estimates the current state of the system, and second, an update step 54 refines the estimates by incorporating the latest measurement. The KF is particularly advantageous in the control device 30 for determining outliers in measured blood glucose levels, as it can effectively filter out noise and, more notably, the analysis of the KF residuals-representing the prediction error with respect to observations-provides an indication of the concordance between measurements and model outputs. This analysis is especially useful for detecting sensor faults, as it allows for the identification of discrepancies that may suggest an outlier, thereby enhancing the reliability of the data set used for generating RC recommendations. For example, the KF may predict the system state by integrating a personalized insulin-to-glucose model, and then refine this prediction by comparing it to the actual measured blood glucose levels, thereby identifying any discrepancies that may indicate an outlier.

In some aspects, the outlier unit 34 may employ various derivations of the KF for the detection of candidate outliers in the measured blood glucose levels. These derivations could be, but are not limited to, Extended KF, Unscented KF, Cubature KF, Square Root KF, Information KF, Ensemble KF, and Particle Filter for example. Each of these KF derivations offers a different approach to processing and analyzing the data, with some being more computationally intensive than others. For instance, the Particle Filter may require the propagation of many particles through the model equations to estimate posterior state distributions, which may be non-Gaussian, but it requires fewer assumptions regarding the linearity of the system and the statistical properties of the noise. The outlier unit 34 may also utilize various models, whether linear or nonlinear, physiological or empirical (blackbox, greybox), to assess the mismatch between the model predictions and the actual measured blood glucose levels (i.e. CGM measurements). This mismatch assessment is a core function of the outlier unit 34, as it helps to identify when the measured blood glucose levels deviate from expected patterns, which may indicate a fault or outlier of the measured blood glucose levels. Depending on the hardware capabilities and the specific requirements of the diabetes management system, a more parsimonious version of the KF, such as the base KF coupled with a linear model, may be preferred. This approach focuses on the ability of the method to capture sudden variations of mismatches between the model and the measurements, which is of paramount concern for sensor fault detection. In contrast, a more accurate state estimation, which might be desired for model-based control applications such as computing insulin from state prediction, would potentially require a more complex KF derivation. The choice of KF derivation and model type is thus informed by the trade-off between computational efficiency and the level of accuracy and robustness desired in the detection of outliers.

According to a preferred embodiment, the KF is configured to predict the system state by integrating a 6th order insulin-to-glucose linear model personalized from user's settings, the model parameters comprising at least:
- a meal ratio (MR); and/or
- an insulin sensitivity factor (ISF); and/or
- an insulin diffusion rate represented by a diffusion time constant (τ_{IOB}); and/or
- a meal digestion rate represented by a digestion time constant (τ_{D}).

Such a characteristic allows the control device 30 to provide a tailored approach to diabetes management by incorporating individual user settings. This personalization ensures that the RC recommendations are based on a model that closely reflects the user's specific physiological response to insulin and carbohydrate intake, leading to more accurate and effective management of blood glucose levels. The use of a 6th order linear model allows for a nuanced representation of the insulin-to-glucose dynamics, which can account for complex interactions over time, thereby enhancing the predictive accuracy of the system. This can be particularly advantageous in preventing hypoglycemic events, as the system can more precisely forecast blood glucose trajectories and suggest timely and appropriate RC interventions. It is also to be noted that the synergistic integration of the meal ratio (MR), insulin sensitivity factor (ISF), insulin diffusion rate (τIOB), and meal digestion rate (τD) within the 6th order insulin-to-glucose linear model facilitates a comprehensive and individualized management of blood glucose levels. By combining these parameters, the control device 30 can more accurately simulate the complex interplay between carbohydrate intake, insulin administration, and their respective rates of action and absorption. This synergy allows for the refinement of the model to reflect the user's specific metabolic patterns, enhancing the precision of the RC recommendations. The combined effect of these parameters ensures that the RC recommendations are not just based on static measurements, but are dynamically adjusted to the user's personalized physiological responses, leading to a more effective and proactive approach to preventing hypoglycemic events.

In the context of the present disclosure, a "6th order insulin-to-glucose linear model" refers to a mathematical representation that characterizes the relationship between insulin administration and glucose levels over time, using a linear state space model approximating actual system dynamics. The model is described by a set of differential equations that represent the dynamics of insulin action and glucose absorption, up to the sixth derivative with respect to time. The 6th order model aims to capture the primary dynamics of the insulin-to-glucose interaction, providing a balance between model complexity and computational efficiency, which can be particularly useful for real-time applications in insulin delivery systems.

As stated above, the use of a 6th order linear model allows for a nuanced representation of the insulin-to-glucose dynamics, which can account for complex interactions over time, thereby enhancing the predictive accuracy of the system. However, a KF configured to predict the system state by integrating a insulin-to-glucose linear model of any order could also produce acceptable results.

In the context of the present disclosure, "meal ratio" MR refers to a parameter used to personalize the insulin-to-glucose model, representing the ratio of the amount of carbohydrates in a meal to the amount of insulin administered to metabolize those carbohydrates. The MR is a factor in determining the insulin dosage for a given carbohydrate intake, and it is used to adjust the model parameters to reflect the user's individual dietary habits and insulin sensitivity. The MR can be set by the user, healthcare provider or determined based on historical data and may be adjusted over time to optimize glycemic control.

In the context of the present disclosure, "insulin sensitivity factor" (ISF) refers to a parameter that quantifies the relationship between insulin administration and its effect on lowering blood glucose levels. The ISF may be used to adjust the dosage of insulin to achieve a desired blood glucose target and is typically personalized based on the user's insulin sensitivity and metabolic response. The ISF can be determined through clinical assessment or derived from historical data of the user's insulin usage and corresponding blood glucose responses.

In the context of the present disclosure, "diffusion time constant" (τIOB) refers to a parameter representing the rate at which insulin diffuses into the bloodstream and begins to lower blood glucose levels after administration. This constant is an integral part of the insulin-to-glucose model, as it helps to predict the time-dependent effects of insulin on blood glucose concentration. The diffusion time constant can be personalized based on the user's physiological characteristics and may vary depending on various factors. By incorporating τ_{IOB} into the model, the control device 30 can more accurately forecast the insulin's impact on blood glucose levels over time, which is particularly useful for timing RC recommendations to prevent or mitigate hypoglycemia.

In the context of the present disclosure, "digestion time constant" (τD) refers to a parameter that characterizes the rate at which ingested carbohydrates are digested and absorbed into the bloodstream as glucose and therefore appear in the measured blood glucose levels. This constant is a component of the insulin-to-glucose model that influences the timing and magnitude of a postprandial glucose response. The digestion time constant can be personalized to reflect the user's individual digestive kinetics, which may be influenced by factors such as meal composition, gastrointestinal motility, and metabolic health. By incorporating τD into the model, the control device 30 can more accurately predict the rise in blood glucose levels following a meal, which is particularly useful for determining the timing and amount of insulin administration or RC recommendations to maintain glycemic control.

In the context of the present disclosure, the model parameters comprising at least a MR and/or a ISF and/or a τIOB and/or a τD means that the model comprises the parameters or derivatives of said parameters.

According to an embodiment, the outlier unit 34 is further configured to identify outliers specifically during a postprandial period. Such a characteristic allows the control device 30 to specifically target the postprandial period, which is a time when blood glucose levels can be particularly volatile, in an upward trend, due to the ingestion of food. By focusing on this period, the outlier unit 34 can more effectively identify readings (i.e. measured blood glucose levels) that do not conform to expected post-meal blood glucose patterns, thereby enhancing the accuracy of the RC recommendations. This targeted approach to outlier detection is especially beneficial for users who may experience unpredictable glycemic responses after meals, as it provides a more refined analysis of blood glucose data during a time when accurate RC recommendations are of utmost relevance for preventing postprandial hypoglycemia. During the postprandial period, particularly close to the time of meal declaration, the control device 30 may adjust an anticipatory rule for predicting the RC recommendation. This adjustment is based on the expectation that glycemia will rise due to the amount of carbohydrates to have been ingested. Consequently, the system can, in some instances, relax the usual anticipatory rule to avoid raising an RC alert that could disrupt the user unnecessarily. This relaxation is maintained up to a point where the user's glycemia reaches a level below which an RC trigger is desired, ensuring that RC alerts are raised when truly warranted. This approach helps to prevent the overcorrection of blood glucose levels, which can be particularly disruptive post-meal when the body is naturally working to assimilate ingested carbohydrates and stabilize glycemia.

According to a preferred embodiment, the outlier unit 34 is further configured to consider the last measured blood glucose level of the measured blood glucose levels to be an outlier if a distance (d) involving an innovation matrix and KF residuals, is inferior to a dynamic threshold (d_{THR}). Such a characteristic allows the control device 30 to dynamically adjust the sensitivity of outlier detection, which is particularly beneficial in scenarios where blood glucose levels may fluctuate rapidly, such as during physical activity or after meal intake. By setting a dynamic threshold d_{THR} that adapts to the current physiological context of the user, the control device 30 / system 10 can more accurately discern between true outliers and legitimate rapid changes in glucose levels. This results in a more robust and reliable RC recommendation process, minimizing the risk of false alerts and ensuring that interventions are based on the true state of the user's glycemic status. The ability to fine-tune outlier detection in real-time enhances the system's utility across a wide range of situations, providing users with a higher degree of confidence in the RC recommendations received.

In the context of the present disclosure, "distance" (d) refers to a metric used to quantify the discrepancy between the predicted state of a system and the actual observed measurements. In other words, d is a measure of how the KF prediction error varies from the expected prediction error. Therefore, the innovation matrix is used to account for the usual variability on the prediction error. An important distance d means that the KF unusually wrongly predicts, which can be symptomatic of a sensor fault. Specifically, it is the Mahalanobis distance, which is a multivariate distance measure that accounts for the correlations among the variables and scales the distance calculation according to the variability of each variable. This distance is calculated from an innovation matrix, which represents the variability on the KF residuals between the predicted and observed measurements, and the KF residuals, which are the discrepancies between the actual measurements and the KF predictions. If the calculated distance d is less than a dynamically determined threshold d_{THR}, the last measured blood glucose level is considered to be an outlier. This approach allows for a more sophisticated outlier detection mechanism that is sensitive to the inherent variability and correlation structure of the data, leading to more accurate and reliable blood glucose level assessments for the purpose of RC recommendation.

The dynamic threshold d_{THR} for outlier detection is computed based on a moving mean or median of d signal values taken over a past window of time. This approach allows for the threshold to adapt to changes in the user's physiological state over time, providing a baseline that is reflective of the user's recent glycemic trends. To further enhance the accuracy of outlier detection, the computation of the moving mean or median may be trimmed to discard the extreme values, such as the peaks that may contain the sought outlier, thereby capturing a more representative baseline of d. An additive offset is also applied to d_{THR}, which can provide a safety margin to account for measurement uncertainties or expected physiological variations. Alternatively, a multiplicative scaling factor may be applied to d_{THR}, allowing d_{THR} to scale proportionally with the variability observed in d. This scaling can be particularly useful in situations where the measured blood glucose levels exhibit greater fluctuation, such as during periods of intense physical activity or stress, ensuring that the outlier detection remains sensitive to true outliers while avoiding false positives.

In some embodiments, the outlier unit 34 may utilize a Statistical Chi-Square Test to assess the goodness of fit between KF residuals and a theoretical distribution, such as a Gaussian distribution. This test is particularly useful for determining whether the observed residuals from the KF deviate in a statistically meaningful way from what would be expected if the system were correctly modeling the underlying physiological process. A substantial deviation, as indicated by a Chi-Square Test result that exceeds a predefined threshold, may suggest that a measured blood glucose level is an outlier. This deviation is defined as reading a larger value on the residuals' distribution's expected value, or statistical mean, than is typically observed in the absence of outliers in the measured blood glucose levels. Such a finding would be indicative of a potential fault in the measured blood glucose levels or an anomaly in the glucose measurement process, prompting the outlier unit 34 to potentially exclude the affected data point from the RC recommendation process. In the context of the present disclosure, a "Statistical Chi-Square Test" is a statistical method used to compare observed data with data expected to be obtained according to a specific hypothesis. The test calculates the chi-square statistic, a measure of the discrepancy between observed and expected frequencies in one or more categories. This statistic is then compared to a chi-square distribution to determine the likelihood of the observed distribution occurring by chance. If the calculated chi-square statistic exceeds the value of the chi-square distribution for a given level of statistical confidence, it suggests that the observed data do not fit the expected distribution, indicating the presence of an outlier or a deviation from the hypothesized pattern.

In some embodiments, machine learning techniques may be employed to enhance the detection of outliers based on the residuals obtained from the KF. These techniques involve training machine learning models on historical data to classify normal and faulty behavior. Algorithms such as Support Vector Machines (SVM), Random Forests, or Neural Networks can be utilized for this purpose. SVMs offer effectiveness in high-dimensional spaces and their ability to model non-linear boundaries due to their kernel trick feature. Random Forests, which consist of multiple decision trees, offer robustness against overfitting and provide a measure of feature importances. Neural Networks, particularly deep learning models, are capable of learning complex patterns and relationships in the data through their layered structure.

The use of these machine learning techniques in classification and clustering contexts allows for a comprehensive analysis of the data, where each technique may capture different aspects or patterns indicative of outliers. By combining these techniques or running them concurrently, the system can leverage a consensus approach to enhance the reliability of outlier detection. For instance, a voting mechanism could be implemented where if a majority of the machine learning algorithms determine the presence of an outlier, the control device 30 may consider bypassing the RC recommendation.

Alternatively, a safer mode could require all algorithms to converge on the detection of an outlier before taking action. This multi-algorithm approach can improve the robustness of the system against false positives and negatives, ensuring that RC recommendations are based on the accurate classification of blood glucose level measurements.

According to an embodiment, In order to enhance the accuracy of RC recommendations, the control device 30 is configured to implement a three-step procedure for managing outliers in the measured blood glucose levels. Initially, the outlier unit 34 is configured to use a KF to analyze the residuals of the blood glucose measurements levels, thereby identifying candidate outliers that deviate from the predicted values. These candidates are then subjected to a series of data-driven confirmation rules, which may include assessing the temporal context of the readings, the physiological plausibility of the glucose trends, and the consistency of the data with known user activities or events. Upon confirmation of the outliers, a control strategy of the RC unit 36 is refined accordingly. This may involve adjusting the RC recommendation to account for the identified outliers, ensuring that the system's response is based on the user's true glycemic status. By systematically determining, confirming, and responding to outliers, the control device 30 provides a robust and reliable approach to managing RC alerts, thereby improving the safety and efficacy of diabetes management.

In an embodiment, a measured blood glucose level of the measured blood glucose levels is considered to be an outlier if it is considered by the outlier unit 34 to be affected by a compression artifact. Such a characteristic allows the control device 30 to enhance the reliability of RC recommendations by identifying and excluding glucose measurements that may be distorted due to physical interference with the glucose monitoring device, wherein said glucose monitoring device is a CGM for example. Compression artifacts can occur when the sensor (i.e CGM) is subjected to pressure, leading to falsely low glucose readings that could trigger unnecessary RC recommendations. By recognizing these artifacts, the control device 30 can prevent inappropriate responses to these inaccurate readings, thereby maintaining the accuracy of the RC recommendations and avoiding potential overtreatment of hypoglycemia. This feature is particularly beneficial for users who are active or sleep on their monitoring device for example, as it ensures that the RC recommendations are based on true glucose values rather than artifacts of the measurement process.

In the context of the present disclosure, a "compression artifact" refers to a type of error in glucose readings that occurs when an external force is applied to the glucose monitoring device, typically a CGM, which results in a temporary deformation of the sensor or its surrounding tissue. This deformation can cause a disruption in the interstitial fluid dynamics or sensor function, leading to a transient and artificial change in the measured glucose level that does not accurately reflect the true blood glucose concentration. Compression artifacts are often characterized by a sudden, unphysiological drop in the recorded glucose levels, followed by a return to more typical readings once the pressure is relieved. These artifacts can be particularly problematic during periods of rest or sleep when the user may be lying on the sensor, or during intense physical activity where the device may be compressed against the body. The ability to accurately identify and exclude readings affected by compression artifacts is an integral part of ensuring the reliability of glucose monitoring systems and the safety of the user, as it helps to prevent inappropriate therapeutic decisions based on erroneous data.

In some aspects, the control device 30 may utilize confirmation rules to verify that a KF outlier candidate is indeed an unphysiological compression artifact. The confirmation process may include a nighttime condition, which focuses on detecting nocturnal compression artifacts to reduce the risk of false positives that could be increased by day-to-day activities. The outlier unit 34 may also be configured to consider the temporal distance to past declared, estimated, or measured physical activities and carbohydrate intakes (i.e amount of carbohydrates ingested by the unique user), as these factors can influence measured blood glucose levels and the likelihood of compression artifacts. A downward residual condition may be applied, where a negative residual value is a negative value which confirms a nadir trend for the outlying period. This condition is particularly relevant during nighttime, as it enhances the safety of detection by reducing the likelihood of false positives. However, this condition may be relaxed in other applications where nighttime detection is not as pertinent. According to the present invention, a "nadir trend" refers to a pattern or trajectory of measured blood glucose levels reaching their lowest point over a given period.

The outlier unit 34 may further be configured to evaluate the first derivative condition, where the current slope of a measured blood glucose level trend is compared to a predefined negative threshold. If the slope is less than this threshold, it confirms a substantial and unusual drop in glycemia, indicative of a potential compression artifact. Additionally, a second derivative condition may be assessed, where a sudden discontinuity in the slope, as compared to a predefined threshold, attests to an unphysiological change in trend. An alternative embodiment for the second derivative condition may involve computing the ratio of two successive first derivative samples and comparing it to a predefined threshold. This approach allows for the detection of rapid changes in the rate of glycemia decrease, which are characteristic of compression artifacts.

Furthermore, a net lOB condition may be implemented, where the net lOB value is expected to be less than zero to enhance the safety of the detection process. This ensures that the sudden drop in measured blood glucose levels cannot be attributed to the effects of past insulin infusion. By incorporating these confirmation rules, the control device 30 can more accurately identify and exclude compression artifacts, thereby improving the reliability of the RC recommendations and ensuring that interventions are based on accurately measured blood glucose levels. In the context of the present disclosure, "net Insulin On Board" or "net IOB" refers to the amount of insulin that has been administered and is still available within the user's body, adjusted for a basal insulin rate. The net lOB is calculated by subtracting the amount of insulin that would have been delivered as part of the user's basal rate from the total IOB. This calculation takes into account the insulin that has been infused to counteract ingested carbohydrates as well as insulin administered to correct high blood glucose levels. The net IOB provides a more accurate reflection of the insulin's potential effect on lowering blood glucose levels at any given moment, as it considers the insulin that is effectively in excess of the basal insulin requirements. In the context of the present disclosure, "basal insulin rate" refers to insulin administered to a user to maintain normal blood glucose levels in the absence of meals. This rate is typically set based on the unique user's basal metabolic insulin requirements, which are the insulin requirements during periods of fasting or in between meals. The basal insulin rate is designed to mimic the steady release of insulin by a healthy pancreas and is often delivered by an insulin pump or through long-acting insulin injections. The basal insulin rate can be adjusted based on various factors such as changes in the unique user's routine, physical activity, stress levels, or other physiological conditions that may affect insulin sensitivity. It is a foundational component of insulin therapy for individuals with diabetes, particularly those on intensive insulin therapy for example. The basal insulin rate is distinct from bolus insulin; which is administered to manage the rise in blood glucose levels due to carbohydrate intake from meals.

In some embodiments, the outlier unit 34 of the control device 30 is configured to analyze the relationships between various features, representing a feature space, such as measured blood glucose levels derivatives, net IOB, Carbohydrate On Board (COB), KF residuals, and additional sensor data like accelerometry or cardiac frequency for example, using a Support Vector Machine (SVM). The SVM allows one to make a hyperplane that effectively separates the feature space into two distinct categories: the outlying period and the physiological period. This separation is instrumental in distinguishing between normal physiological fluctuations and potential outliers that may indicate a fault in the measured blood glucose levels or an unphysiological event affecting the glucose readings. In the context of the present disclosure, a "hyperplane" is a geometric concept that generalizes the notion of a plane in higher dimensions. Specifically, in a feature space of n dimensions, a hyperplane is a flat affine subspace of dimension n-1, which means it is one dimension less than the feature space itself. For example, in a three-dimensional space, a hyperplane would be a two-dimensional plane. In the context of machine learning and specifically in the use of SVM, a hyperplane is used to separate different classes of data points by finding the plane that maximizes the margin between the classes. The hyperplane is defined by a set of weights and a bias, which are optimized during the training of the SVM to create the decision boundary that can classify new data points based on their position relative to the hyperplane.

In another embodiment, the outlier unit 34 may utilize a trained Random Forest method to define a threshold surface within the feature space. The Random Forest method, which consists of an ensemble of decision trees, can classify the measured blood glucose levels as either outliers or non-outliers based on the aforementioned features. This method is particularly advantageous due to its ability to handle high-dimensional data and its robustness against overfitting, which is beneficial for the accurate detection of outliers in the measured blood glucose levels.

Further, in some cases, the outlier unit 34 may implement Neural Networks, including Convolutional Neural Networks (CNNs) or Recurrent Neural Networks (RNNs), for the classification of outliers. These types of Neural Networks are adept at capturing complex patterns and temporal dependencies in the data, which can be particularly useful for identifying subtle or transient anomalies in the measured blood glucose levels that may not be readily apparent through other methods.

Additionally, the outlier unit 34 incorporates Gaussian Process Regression (GPR) to model the relationship between input features and the output classification. GPR provides not just predictions for classification but also uncertainty estimates, which are of great value when defining a threshold surface with confidence intervals. These uncertainty estimates can enhance the decision-making process by providing a probabilistic assessment of the data, thereby allowing for more informed and cautious interventions in the management of blood glucose levels.

The outlier unit 34 is configured to consider a measured blood glucose level to be affected by a compression artifact based at least on a deviation from a predicted glycemia and a measured blood glucose level. Such a configuration allows the control device 30 to discern between genuine fluctuations in measured blood glucose levels and those affected by compression artifacts. By comparing the deviation between predicted glycemia and actual measured blood glucose levels, the outlier unit 34 can effectively identify when a reading is likely to have been compromised. This ensures that the RC recommendations are based on accurate and reliable data, particularly in situations where the CGM sensor might be under pressure, such as during sleep or physical activity. The ability to filter out these specific types of outliers can prevent unnecessary or incorrect carbohydrate intake recommendations, thereby optimizing the user's glycemic management and reducing the risk of hypoglycemia. This feature is especially advantageous for maintaining the integrity of the RC recommendation process, ensuring that users receive alerts and interventions that accurately reflect their physiological state.

In accordance with the present disclosure, the outlier unit 34 of the control device 30 is configured to utilize a predictive model that takes into account both the predicted glycemia and the actual measured blood glucose levels. The predictive model may employ various algorithms and techniques to forecast the expected glycemia based on a range of factors, including but not limited to, historical blood glucose data, recent trends in the unique user's measured blood glucose levels, and unique user's physiological parameters. When the outlier unit 34 detects a deviation between the predicted glycemia and the actual measured blood glucose level that exceeds a predefined threshold, it considers this an indication of a potential compression artifact. This threshold may be dynamically adjusted based on the user's current state and the variability of their blood glucose measurements. By setting this threshold, the control device 30 can distinguish between normal physiological fluctuations in blood glucose levels and those readings that are likely to have been affected by external pressure on the glucose monitoring device. This feature is particularly useful in scenarios where the user is engaged in activities that may exert pressure on the CGM, such as sleeping or exercising. The ability to accurately identify and disregard these affected readings helps to maintain the integrity of the RC recommendation process, ensuring that the user is provided with reliable and actionable information for managing their diabetes.

The control device 30 comprises an RC unit 36. The RC unit 36 is configured to estimate an RC recommendation using slopes of the measured blood glucose levels. The RC unit 36 function of estimating an RC recommendation using slopes of the measured blood glucose levels allows a dynamic assessment of the user's glucose trends. This enables the control device 30 to anticipate potential hypoglycemic events and recommend appropriate interventions in a timely manner, potentially reducing the risk of severe hypoglycemia for example.

The RC unit 36 is further configured to estimate an RC recommendation if the most recent measured blood glucose levels of the measured blood glucose levels show a deceleration. Such a characteristic allows the control device 30 to provide RC recommendations that are not just reactive to current blood glucose levels, but also predictive of future trends. By analyzing the deceleration in blood glucose levels, the RC unit 36 can identify when the rate of decrease is slowing down, which may indicate an impending stabilization or potential reversal of the downward trend. This preemptive capability can be particularly advantageous for preventing hypoglycemia, as it allows for earlier intervention with RC when the blood glucose levels are still within a safe range, rather than waiting for them to reach a level that is already indicative of hypoglycemia. This proactive approach can improve the user's quality of life by reducing the frequency and severity of hypoglycemic episodes, and can also minimize the cognitive burden on the user by reducing the number of decisions they have to make regarding their glucose management.

The RC unit 36 is configured to adjust the RC recommendation based on the physical activity data. Such a configuration allows the control device 30 to account for the impact of physical activity on measured blood glucose levels, which is a dynamic factor in diabetes management. The inclusion of physical activity data in the RC recommendation process enables the control device 30 to provide more personalized and context-aware recommendations. By adjusting the RC recommendation based on the user's physical activity, the system can better anticipate the physiological effects of exercise, which often include increased insulin sensitivity and glucose uptake by muscles, potentially increasing the risk of exercise-induced hypoglycemia. This feature enhances the control device 30 ability to tailor RC recommendations to the unique user's lifestyle, promoting more effective and safer diabetes management. Additionally, it supports users in maintaining an active lifestyle by providing them with the confidence that their glucose levels are being monitored and managed with consideration of their physical activities.

The control device 30 comprises a predictive unit 40, the predictive unit 40 being configured to compute at least a predicted glycemia at least based on the user data. Such a characteristic allows the control device 30 to enhance the management of diabetes by providing foresight into the user's glycemic future. The predictive unit 40 ability to compute predicted glycemia based on user data enables the control device 30 to forecast potential hypoglycemic events before they occur, allowing for preemptive measures to be taken. This predictive capability can lead to improved glycemic control, as it provides users and healthcare providers with valuable information to make informed decisions regarding carbohydrate intake and insulin dosing. Additionally, the predictive unit 40 use of user data for forecasting purposes ensures that the predictions are personalized and tailored to the individual's metabolic responses, further increasing the accuracy and relevance of the RC recommendations. This can result in a reduction of hypoglycemic episodes, enhanced patient safety, and an overall improvement in the quality of life for individuals with diabetes. In the context of the present disclosure, a "predictive unit" 40 refers to a component within the control device 30 that is responsible for calculating future physiological states, such as predicted glycemia levels, based on current and historical user data. The predictive unit may utilize various mathematical models, algorithms, and data processing techniques to analyze trends and patterns in the unique user's physiological data, thereby enabling the control device 30 to anticipate future changes in blood glucose levels. For example, the predictive unit 40 may employ a linear regression model to forecast blood glucose levels based on recent measurements and known rates of glucose absorption and insulin action. In another aspect, the predictive unit 40 might use machine learning algorithms, such as neural networks or support vector machines, to analyze complex datasets and identify subtle correlations that can improve the accuracy of its predictions. Additionally, the predictive unit 40 could incorporate time-series analysis to detect cyclical patterns in blood glucose fluctuations, which can be influenced by daily activities, meal times, and sleep cycles. These examples illustrate the versatility of the predictive unit 40 in adapting to the individualized characteristics of the unique user's metabolic responses and lifestyle factors, thereby enhancing the personalized management of diabetes. In some embodiments, the predictive unit may also employ Model Predictive Control (MPC) strategies to enhance the prediction of future physiological states. MPC is a form of control algorithm that involves creating a model of the system to predict future outcomes and adjusting control inputs accordingly. In the context of glycemia management, MPC can be particularly useful for taking into account the delayed effect of insulin delivery and carbohydrate absorption on blood glucose levels. By considering a range of possible future scenarios and the likely impact of different insulin dosing strategies, the predictive unit can optimize the control of blood glucose levels over a specified prediction horizon, thereby contributing to more stable and precise diabetes management.

The RC unit 36 is configured to estimate the RC recommendation if a predicted glycemia is less than a predicted glycemia threshold (θ1), the current glycemia is less than a current glycemia threshold (θ2), and an Insulin On Board (lOB) is greater than an lOB threshold (θ3). Such a characteristic allows the control device 30 to provide a more nuanced and responsive approach to managing blood glucose levels, particularly in the context of insulin therapy. By incorporating multiple thresholds that take into account predicted glycemia, current glycemia, and IOB, the RC unit 36 can make more informed decisions about when to recommend carbohydrate intake. This multi-faceted analysis ensures that RC recommendations are made at the appropriate times, reducing the risk of over-or under-treatment of hypoglycemia. The integration of these thresholds allows for a more personalized and precise management of diabetes, which can lead to better overall glycemic control and a reduction in the incidence of hypoglycemic events. This proactive strategy can improve patient outcomes and may decrease the long-term complications associated with diabetes. In the context of the present disclosure, "Insulin On Board" (IOB) refers to the amount of insulin that has been administered and is still available within the unique user's body. The IOB is a dynamic value that reflects the insulin that has yet to exert its full glucose-lowering effect. Estimating the lOB involves calculating the remaining available insulin from previous doses, taking into account the time since administration and the known pharmacokinetics of the insulin preparation used. The lOB can be influenced by factors such as the type of insulin, the dosage, and the unique user's insulin sensitivity. For instance, rapid-acting insulin will have a different lOB profile compared to long-acting insulin. The lOB is an integral part of the decision-making process in insulin management, as it helps to prevent insulin stacking and the risk of hypoglycemia. In some embodiments, the control device 30 is configured to estimate the lOB by utilizing a decay function that models the progressive absorption effect of insulin over time. This estimation allows the RC unit 36 to adjust the RC recommendation by considering the residual impact of previously administered insulin on current and predicted glycemia levels.

According to an embodiment, the RC unit 36 is configured to dynamically adjust the predicted glycemia threshold (θ1), the current glycemia threshold (θ2), and the IOB threshold (θ3) based on real-time data and the user's current physiological state. For example, the thresholds may be adjusted to account for factors such as physical activity, stress, illness, or changes in medication. This dynamic adjustment allows the control device 30 to provide RC recommendations that are tailored to the user's immediate situation, thereby enhancing the safety and efficacy of the insulin therapy.

Additionally, the RC unit 36 may incorporate safety features that prevent the recommendation of RC when it is not indicated for safety or user's disturbance reason. For instance, if the predicted glycemia is above θ1 and the current glycemia is above θ2, the RC unit 36 may withhold the RC recommendation even if the lOB is greater θ3. This safety mechanism ensures that RC is recommended conservatively and in accordance with the user's actual glycemic status, minimizing the risk of unnecessary carbohydrate intake and potential subsequent hyperglycemia.

The RC unit 36 is configured to compute the RC recommendation based on a distance between a glycemia target and a linearly forecasted glycemia for a predefined prediction horizon, and wherein said distance is converted to carbohydrate units via a sugaring ratio. Such a configuration allows the control device 30 to provide a precise and personalized RC recommendation by quantifying the exact carbohydrate amount that is likely to bring the unique user's blood glucose level back to a desired target range. By calculating the distance between the glycemia target and the linearly forecasted glycemia, the RC unit 36 can determine the magnitude of intervention that is appropriate for the specific situation. The conversion of this distance into carbohydrate units via a sugaring ratio simplifies the process for the user, translating complex data into actionable and understandable guidance. This approach not just aids in the immediate correction of hypoglycemia but also contributes to the long-term stability of blood glucose levels by providing tailored recommendations that are directly aligned with the unique user's physiological requirements. The ability to forecast glycemia and convert it into a tangible RC recommendation empowers users to manage their condition with greater confidence and precision, potentially reducing the frequency of hypoglycemic episodes and enhancing overall quality of life.

In the context of the present disclosure, a "sugaring ratio" is calculated based on the relationship between the amount of carbohydrates (CHO) that is expected to raise the blood glucose level to a target range and the anticipated increase in blood glucose level over a predefined prediction horizon. The sugaring ratio is estimated using a formula that takes into account the unique user's Body Weight (BW) and a standard absorption factor, which is typically derived from empirical data. For instance, the sugaring ratio may be estimated from the formula sugRatio = 20 BW / (0.6 80) in [gCHO/g/L], where BW is in kilograms. This ratio is then used to convert the distance between a predicted glycemia and a glycemia target into carbohydrate units, which represents the recommended amount of carbohydrates to be ingested to correct or prevent hypoglycemia. The sugaring ratio thus serves as a conversion factor that translates the anticipated change in blood glucose level into an equivalent amount of carbohydrates, facilitating the computation of a personalized RC recommendation.

The RC unit 36 is further configured to adjust the RC recommendation based at least on IOB, such that an adjusted RC recommendation is an increasing function of the IOB. Such a configuration allows the control device 30 to dynamically tailor the RC recommendation in accordance with the amount of insulin that is still available within the unique user's body, known as lOB. By considering IOB, the RC unit 36 can more accurately determine the amount of carbohydrates that the unique user may require to prevent or treat hypoglycemia. This is particularly advantageous as it accounts for the insulin's ongoing glucose-lowering effect, which can vary depending on factors such as the type of insulin used, the time since administration, and the unique user's insulin sensitivity. The ability to adjust the RC recommendation as an increasing function of the IOB ensures that the user is not advised to consume more carbohydrates than is actually necessary based on their current physiological state, thereby avoiding potential hyperglycemia. This feature enhances the precision of the RC recommendation, leading to more effective and safer blood glucose management, and ultimately contributes to the unique user's overall well-being and metabolic stability.

An RC recommendation based at least on lOB corresponds to a recommendation that is based on the net IOB, ensuring that the RC unit 36 accounts for the dynamic insulin levels when determining the appropriate amount of rescue carbohydrates to recommend for preventing or treating hypoglycemia. This approach allows for a more personalized and precise RC recommendation, as it aligns the recommendation with the unique user's current physiological insulin profile.

The control device 30 comprises a transmission unit 38. The transmission unit is configured to transmit the RC recommendation. The transmission unit is configured to transmit the RC recommendation if and only if the last measured blood glucose level of the measured blood glucose levels is not considered to be an outlier. The transmission unit 38 conditional operation, which transmits the RC recommendation if and only if the last measured blood glucose level is not considered to be an outlier, ensures that the recommendations provided to the user are based on reliable and accurate data. This selective transmission acts as a safeguard against providing potentially harmful advice based on erroneous readings.

The transmission unit 38 is configured to transmit the RC recommendation to another device, such as a mobile phone, a smartwatch, a dedicated receiver, or a healthcare provider's monitoring system. This transmission can occur over various communication protocols, including but not limited to Bluetooth, Wi-Fi, cellular networks, or near-field communication (NFC). The ability to transmit the RC recommendation to another device enables the user or healthcare provider to receive timely alerts and take appropriate action, thereby enhancing the user's safety and the efficacy of diabetes management. The transmission unit 38 is also configured to send an alert to the user or a designated recipient when the RC recommendation is transmitted. The alert may serve to notify the user or recipient of the RC recommendation, prompting them to take action based on the recommendation provided. The alert could be in the form of a visual notification, an audible alarm, a vibration, or any combination thereof, depending on the user's preferences and the capabilities of the receiving device. This feature enhances the responsiveness of the user or caregiver to potential hypoglycemic events, contributing to improved safety and proactive diabetes management.

According to an embodiment, when a measured blood glucose level of the measured blood glucose levels is considered to be an outlier, the transmission unit is configured to not transmit RC recommendation until a bypass period. This bypass period is maintained until at least one of the measured blood glucose levels of the measured blood glucose levels has recovered and is not considered to be an outlier. The end of the bypass period is based on two conditions: either the time has reached or exceeded the nadir time plus a predetermined upward time, or the measured blood glucose level has risen to or above a certain level. According to an embodiment, the bypass period is designed with safety exceptions to ensure unique user well-being. Should the most recently measured blood glucose level fall below a safety threshold, such as 55 mg/dL for example, or if the bypass period extends beyond a maximum duration, the transmission unit is configured to be able to transmit RC recommendation again. Such a configuration allows the control device 30 to prevent the risk of actual hypoglycemia that may not be detected due to a measured blood glucose level considered to be an outlier.

According to an embodiment, the control device 30 is configured to assess a blood glucose level prediction at a time ahead of the bypass period, up to a predefined prediction horizon. If this blood glucose level prediction is below a predefined hypoglycemia safety threshold, the bypass period will be reduced to zero, as this indicates a genuine risk of hypoglycemia. Such a configuration allows the control device 30 to prevent the risk of actual hypoglycemia. For the purpose of making these assessments, the control device 30 may be configured to employ any suitable prediction means such as linear models, nonlinear models, or machine learning algorithms. The choice of prediction means can be tailored to the specific requirements of the diabetes management system, taking into account factors such as computational efficiency, prediction accuracy, and the user's individual physiological response patterns.

The invention also relates to a method for determining an amount of RC recommendation. The method is implemented by the control device 30 as described above and comprises the steps of:
retrieving 50 the user data;
predicting 52, predicting or estimating a current state of the system;
updating 54, updating or refining the estimates by incorporating the latest measured blood glucose level;
determining 56 the RC recommendation at least using slopes of the measured blood glucose levels; and
transmitting the RC recommendation 58.

The step of transmitting the RC recommendation 58 is executed if and only if the last measured blood glucose level of the measured blood glucose levels is not considered to be an outlier based on the predicting 52 and updating 54 steps as described above.

The embodiments, technical effects, and definitions disclosed herein with respect to the control device 30 are also applicable to the method described herein. The method encompasses steps that fully utilize the functionalities and features of the control device 30 described herein. Therefore, all embodiments, technical effects, and definitions pertaining to the device, including but not limited to the evolution of the ISF over time and how it is calculated, are equally applicable to the method. This ensures a comprehensive and unified understanding of both the control device 30 and method aspects of the invention, facilitating the implementation and utilization of the disclosed technology across a range of applications.

The invention also relates to a computer program comprising instructions to cause the control device 30 as described above to execute the steps of the method as described above.

## Claims

1. A control device (30) for determining an amount of carbohydrate (RC) recommendation, the control device comprising:
• a retrieving unit (32), the retrieving unit (32) being configured to retrieve user data, each data of the user data having a timestamp and the user data being related to a unique user, the user data comprising at least:
∘ an amount of insulin infused to the unique user;
∘ an amount of carbohydrates ingested by the unique user;
∘ a plurality of physiological values of the unique user, the plurality of physiological values of the unique user comprising at least measured blood glucose levels;
• an outlier unit (34), the outlier unit (34) being configured to determine whether a measured blood glucose level of the measured blood glucose levels is considered to be an outlier or not;
• an RC unit (36), the RC unit (36) being configured to estimate an RC recommendation using mathematical functions of the measured blood glucose levels; and
• a transmission unit (38), the transmission unit (38) being configured to transmit the RC recommendation;
wherein the transmission unit (38) is configured to transmit the RC recommendation if and only if the last measured blood glucose level of the measured blood glucose levels is not considered to be an outlier.

2. The control device (30) according to claim 1, wherein the outlier unit (34) is configured to determine outliers using a Kalman Filter (KF).

3. The control device (30) according to claim 2, wherein the KF is configured to predict the system state by integrating a 6th order insulin-to-glucose linear model personalized from user's settings, the model parameters comprising at least:
• a meal ratio (MR); and/or
• an insulin sensitivity factor (ISF); and/or
• an insulin diffusion rate represented by a diffusion time constant (τ_{IOB}); and/or
• a meal digestion rate represented by a digestion time constant (τ_{D}).

4. The control device (30) according to claim 3, wherein the outlier unit (34) is further configured to consider the last measured blood glucose level of the measured blood glucose levels to be an outlier if a distance (d) involving an innovation matrix and KF residuals, is inferior to a dynamic threshold (d_{THR}).

5. The control device (30) according to any of the claims 1 to 4, wherein the outlier unit (34) is further configured to identify outliers specifically during a postprandial period.

6. The control device (30) according to any of the claims 1 to 5, wherein the RC unit (36) is further configured to estimate an RC recommendation if the most recent measured blood glucose levels of the measured blood glucose levels show a deceleration.

7. The control device (30) according to any of the claims 1 to 6, wherein the control device (30) comprises a predictive unit (40), the predictive unit (40) being configured to compute at least a predicted glycemia at least based on the user data.

8. The control device (30) according to claim 7, wherein the RC unit (36) is configured to estimate the RC recommendation if a predicted glycemia is less than a predicted glycemia threshold (θ1), the current glycemia is less than a current glycemia threshold (θ2), and an Insulin On Board (lOB) is greater than an IOB threshold (θ3).

9. The control device (30) according to any preceding claim, wherein a measured blood glucose level of the measured blood glucose levels is considered to be an outlier if it is considered by the outlier unit (34) to be affected by a compression artifact.

10. The control device (30) according to claims 7 and 9, wherein the outlier unit (34) is configured to consider a measured blood glucose level of the measured blood glucose levels to be affected by a compression artifact based at least on a deviation from a predicted glycemia and a measured blood glucose level.

11. The control device (30) according to any of the claims 1 to 10, wherein the retrieving unit (32) is further configured to retrieve user data including physical activity data of the user, and wherein the RC unit (36) is configured to adjust the RC recommendation based on the physical activity data.

12. The control device (30) according to any of the claims 1 to 11, wherein the RC unit (36) is configured to compute the RC recommendation based on a distance between a glycemia target and a linearly forecasted glycemia for a predefined prediction horizon, and wherein said distance is converted to carbohydrate units via a sugaring ratio.

13. The control device (30) according to claim 12, wherein the RC unit (36) is further configured to adjust the RC recommendation based at least on the IOB, such as an adjusted RC recommendation is an increasing function of the IOB.

14. A method for determining an amount of RC recommendation, the method being implemented by the control device (30) of claim 1 and comprising the steps of:
• retrieving (50) the user data; determining (56) the RC recommendation at least using mathematical functions of the measured blood glucose levels; and
• transmitting the RC recommendation (58);
wherein the step of transmitting the RC recommendation (58) is executed if and only if the last measured blood glucose level of the measured blood glucose levels is not considered to be an outlier.

15. A computer program comprising instructions to cause the control device (30) of claim 1 to execute the steps of the method of claim 14.
